# EUROPEAN PATENT APPLICATION

(11) **EP 3 888 563 A1**
(43) Date of publication of application: **06.10.2021**
(21) Application number: 20167393.6
(22) Date of filing: 31.03.2020
(51) Int. Cl.: A61B 17/00, A61B 90/00, A61B 34/20

(54) **METHOD AND SYSTEM FOR GENERATING IMAGES FOR A FLUOROSCOPY-BASED NAVIGATION SYSTEM**

(71) Applicant: Ecential Robotics, 38610 Gieres (FR)
(72) Inventor: LAVALLÉE, Stéphane, 38140 Saint-Martin-d'Uriage (FR); ARMAND, David, 38120 Saint-Egrève (FR)
(74) Representative: Regimbeau

(57) **Abstract**

The present invention relates to a method and a data processing system for generating navigable images of at least one region of interest ROI of a patient for a fluoroscopy-based navigation system using an X-ray imaging system, a localization system and an imaging kit, said imaging kit being configured to allow images registration in a preferred referential and tracking of surgical tools.

## Description

### FIELD OF INVENTION

The present invention pertains to the field of methods and systems for the generation of images suitable to be use by a fluoroscopy-based navigation system. In particular, the invention relates to an imaging kit and a method, and system to implement said method, for the generation of images of a region of interest of a patient where the position of each point of the region of interest represented in the image is known in a preferred coordinate system.

### BACKGROUND OF INVENTION

During orthopedic and trauma surgery, fluoroscopy-based navigation system allows the tracking of surgical instruments and the superposition in real time of their contour onto fluoroscopy images. In view of carrying out fluoro-navigation, the instruments used during the surgical intervention are equipped with a tracker coupled to a localization system. Another tracker is mounted onto the patient and is also seen by the localization system. This enables the surgeon to know precisely the position of the surgical instruments at any time of the procedure without need to take additional X-ray image.

However, the tracker attached to the patient in proximity of the surgical field can obstruct the gestures of the surgeon, the movements of an automated surgical robot arm or mask surgical entry points during minimally invasive or open surgeries.

The invention of the present invention provides a solution to overcome such a drawback.

### SUMMARY

The present invention relates to an imaging kit for a fluoroscopy-based navigation system including a localization system, said imaging kit comprising:
- a registration-tracking kit comprising:
   - at least one registration phantom comprising a fiducials support of substantially radiotransparent material having a plurality of radiopaque fiducials and being configured to be attached to a bone a patient;
   - a reference tracker being configured to be attached to a bone the patient;
- at least one implantable element configured to be implanted inside a patient and at least one implant tracker, the implantable element and the implant tracker having each cooperating fixation means configured to movably attach the at least one implant tracker to the at least one implantable element;
wherein the reference tracker and the implant tracker are configured to be tracked by the localization system.

The imaging kit of the present invention advantageously allows to position the registration phantom in the center of a region of interest to be acquired by the x-ray imaging system (i.e. surgical field), while fixing the reference tracker in proximity of said the region of interest. Advantageously, the reference tracker may be used to navigate surgical tools to implant the at least one implantable element inside the patient. Since the implantable element is configured to carry itself an implant tracker, the reference tracker, as the registration phantom may be remove advantageously leaving more space to the surgeon and the medical stuff to perform surgical acts inside the ROI.

According to one embodiment, the cooperating fixation means comprise complementary magnetic elements configured to maintain the at least one implant tracker and the at least one implantable element attached together.

According to one embodiment, the registration-tracking kit further comprises a base made of a substantially radiotransparent material and being configured to be rigidly fixed to a bone of the patient; wherein said base comprises at least one base fixation element, the registration phantom comprises at least one phantom fixation element and the reference tracker comprises a tracker fixation element, said base fixation element, phantom fixation element and tracker fixation element being configured to attach, either separately or together, the reference tracker and the registration phantom to the base. This embodiment advantageously allows to position the registration phantom in the center of a region of interest to be acquired by the x-ray imaging system (i.e. surgical field), while fixing the base on a body part of the patient in proximity of said the region of interest. Advantageously, since the base does not need to be fixed inside the surgical field, the surgeon will have a clear surgical site allowing him to perform a greater variability of acts. Furthermore, the invention advantageously allows to fix the base on a more easily accessible anatomic part of the patient.

According to one embodiment, the imaging kit comprises two implantable elements and one implant tracker for each implantable element, wherein each implantable element is a pedicle screw configured to be implanted into a vertebra. This embodiment may be advantageously used during surgery to correct deformity, and/or treat trauma or to immobilize part of the spine to assist fusion by holding bony structures together.

According to one embodiment, the implantable element is a joint prosthesis.

According to one embodiment, the imaging kit comprises at least two implantable elements, each implantable element being a bone screw configured to be implanted into a bone of a joint.

The present invention further relates to a method for generating of navigable images of at least one region of interest ROI of a patient for a fluoroscopy-based navigation system using an X-ray imaging system, an imaging kit according to any one of the embodiments hereabove and a localization system configured to track the reference tracker and the at least one implant tracker of the imaging kit, said method comprising the following steps:
- receiving at least one set of 2D X-ray images of at least one portion of the ROI, wherein the registration phantom had been previously attached to a patient's bone so that the registration phantom is at least partially comprised in the ROI during the acquisition of the set of 2D X-ray images, such that said set of 2D images comprises at least two 2D images containing each at least one detectable radiopaque fiducial of the registration phantom;
- receiving information on the position of the fiducials support and the position of the reference tracker in a patient coordinate system fixed with respect to the patient's bone to which is attached the registration phantom;
- generating at least one registered image by registering the set of 2D images in the patient coordinate system using the known position of the fiducials support in said patient coordinate system;
- receiving a first set of data, obtained using the localization system, comprising data on position and 3D spatial orientation of the reference tracker, said reference tracker previously attached to a bone of the patient in proximity of the registration phantom, and position and 3D spatial orientation of the at least one first implant tracker attached to the at least one first implantable element, said at least one first implantable element previously rigidly fixed to a body part of the patient;
- using the first set of data obtained from the localization system to compute a first rigid transformation between the patient coordinate system and a coordinate system of the at least one first implant tracker;
- generating at least one navigable image by applying the first rigid transformation to the at least one registered image so that each point of the ROI in the at least one navigable image has a known position in the coordinate system of the at least one first implant tracker.

The present invention also relates to a computer program comprising instructions, which when the program is executed by a computer, causes the computer to carry out the steps of the method according to any one of the embodiments described hereabove.

The present invention also relates to a computer-readable storage medium comprising instructions that when executed by a computer, causes the computer to carry out the steps of the method according to any one of the embodiments described hereabove.

The present invention relates as well to a data processing system for generating navigable images of at least one region of interest ROI of a patient for a fluoroscopy-based navigation system using an X-ray imaging system, an imaging kit according to any one of the embodiments described hereabove and a localization system, said localization system being configured to track the reference tracker and the at least one implant tracker of the imaging kit, said data processing system comprising:
- an input module configured to receive at least one set of 2D X-ray images of at least one portion of the ROI, wherein the registration phantom had been previously attached to a patient's bone so that the registration phantom is at least partially comprised in the ROI during the acquisition of the set of 2D X-ray images, such that said set of 2D images comprises at least two 2D images containing each at least one detectable radiopaque fiducial of the registration phantom; said input module being further configured to receive information on the position of the fiducials support and the reference tracker in a patient coordinate system fixed with respect to the patient's bone to which is attached the registration phantom;
- a registration module configured to generate at least one registered image by registering the set of 2D images in the patient coordinate system using the known position of the fiducials support in the patient coordinate system;
- a transformation calculation module configured to:
   - receive a first set of data obtained using the localization system comprising data on position and 3D spatial orientation of the reference tracker, being previously attached to a bone of the patient in proximity of the registration phantom, and data on position and 3D spatial orientation of the at least one first implant tracker attached to the at least one first implantable element, said the at least one first implantable element being previously rigidly fixed to a body part of the patient;
   - use the first set of data to compute a first rigid transformation between the patient coordinate system and a coordinate system of the at least one first implant tracker;
   - generate at least one first navigable image by applying the first rigid transformation to the at least one registered image so that each point of the ROI in the at least one first navigable image has a known position in the coordinate system of the at least one first implant tracker.

According to one embodiment, the registration module is further configured to compute at least one 3D image within the patient coordinate system using at least a part of the set of 2D images.

According to one embodiment, the transformation calculation module is further configured to:
- receive a second set of data obtained from the localization system with a second implant tracker attached to a second implantable element, previously fixed on a body part of the patient, said second set of data comprising data on position and 3D spatial orientation of the reference tracker and 3D spatial orientation of the second implant tracker;
- use the second set of data obtained from the localization system to compute a second rigid transformation between the patient coordinate system and a coordinate system of the second implant tracker;
- generate at least one second navigable image by applying the second rigid transformation to the at least one registered image so that each point of the ROI in the at least one second navigable image has a known position in the coordinate system of the at least one second implant tracker.

According to one embodiment, the data processing system further comprises a visualization module configured to receive a user instruction selecting one among the first rigid transformation or the second rigid transformation to be applied to the at least one registered image.

According to one embodiment, the data processing system further comprises a dynamic image correction module configured to:
- select the first implant tracker as fixed reference;
- define a first subregion of interest comprising the first implant tracker and a second subregion of interest comprising the second implant tracker for the at least one first navigable image;
- receiving a third set of data obtained using the localization system comprising data on position and 3D spatial orientation of the first implant tracker and the second implant tracker as a function of time;
- use the third set of data to compute a rigid transformation as a function of time between the coordinate system of the first implant tracker and the coordinate system of the second implant tracker;
- apply the rigid transformation as a function of time to the second subregion of interest of the at least one first navigable image so as to dynamically correct during time said navigable image for any drift of the relative position between the first implant tracker and the second implant tracker.

According to one embodiment, the first and second subregions of interest are defined on the at least one first navigable image using a segmentation algorithm or by a manual input of the user.

According to one embodiment, each subregions of interest comprises a vertebra of the patient, and the first and second implant element are pedicle screws fixed to each of the vertebra.

According to one embodiment, each subregions of interest comprises each a portion of a joint of the patient, and the first and second implantable elements are bone screws configured to be fixed to said portions of the joint.

According to one embodiment, the set of 2D images comprises at least one 2D image being an anteroposterior view of the ROI and at least one 2D image being a lateral view of the ROI.

According to one embodiment, the data processing system further comprises a navigation module configured to navigate at least one surgical instrument, comprising a tracker, in the ROI using the at least one navigable image.

### DEFINITIONS

In the present invention, the following terms have the following meanings:
- **"Coordinate system"** refers to a system that uses one or more numbers, or coordinates, to uniquely determine the position of the points or other geometric elements on a manifold such as Euclidean space.
- **"Fluoroscopic navigation"** or **"fluoro-navigation"** refers to a technic of localization of the position of a surgical instrument relative to the anatomy of the patient. By superimposing the instrument's geometry onto fluoroscopic images (i.e. X-ray images), the surgeon can follow live the progression of the intervention.
- **"Image registration"** refers to a process of transforming different sets of data into one coordinate system.
- **"Patient"** refers to a mammal, preferably a human. In the sense of the present invention, a subject may be a patient, i.e. a person receiving medical attention, undergoing or having underwent a medical treatment, or monitored for the development of a disease.
- **"Rigid transformation"** refers to a geometric transformation of a Euclidean space that preserves the Euclidean distance between every pair of points. The rigid transformations include rotations, translations, reflections, or their combination.

### BRIEF DESCRIPTION OF THE DRAWINGS

Features and advantages of the invention will become apparent from the following description of embodiments of a system, this description being given merely by way of example and with reference to the appended drawings in which:
**Figure 1** schematically illustrates the base rigidly fixed to a patient's bone while the registration phantom and the tracker are fixed on the base and the implantable element is rigidly fixed to a patient's bone on which is attached the implant tracker, according to one embodiment.
**Figure 2a** and **2b** are a front and rear prospective view of one registration phantom and **2c** is a front view of the base, according to one embodiment.
**Figure 3a** and **3b** are a front and rear prospective view of one registration phantom, according to one embodiment wherein the registration phantom comprises an elongated member.
**Figure 4** is a block diagram representing the main steps of the method of the present invention according to one embodiment of the invention.
**Figure 5** schematically illustrates the base rigidly fixed to a long bone of the patient while the registration phantom and the tracker are fixed on the base and the implantable element, carrying an implant tracker, is rigidly fixed to the same long bone in proximity of the registration phantom, according to one embodiment.
**Figure 6a** schematically illustrates the elements of the registration-tracking kit attached to one vertebra of a spine and two implantable elements, carrying each an implant tracker, fixed on two nearby vertebrae while **Figure 6b** shows the same configuration as **Figure 6a** after removal of the elements of the registration-tracking kit, according to one embodiment.
**Figure 7** is a block diagram representing the steps of the method of the present invention according to one embodiment of the invention.
**Figure 8a** schematically illustrates the elements of the registration-tracking kit attached to one vertebra on the central portion of a spine and two implantable elements attaching each an implant tracker fixed on two distal vertebrae of the spine while **Figure 8b** shows the same configuration as **Figure 8a** after removal of the elements of the registration-tracking kit, according to one embodiment.
**Figure 9** schematically illustrates two implantable elements, carrying each one implant tracker, rigidly fixed on the femur and one on the pelvis of a patient, according to one embodiment.

### DETAILED DESCRIPTION

The following detailed description will be better understood when read in conjunction with the drawings. For the purpose of illustrating, the imaging kit is shown in the preferred embodiments. It should be understood, however that the present invention is not limited to the precise arrangements, structures, features, embodiments, and aspect shown. The drawings are not drawn to scale and are not intended to limit the scope of the claims to the embodiments depicted. Accordingly, it should be understood that where features mentioned in the appended claims are followed by reference signs, such signs are included solely for the purpose of enhancing the intelligibility of the claims and are in no way limiting on the scope of the claims.

The invention is carried out in the of context of fluoroscopy-based navigation surgery so as to allow an efficient and accurate navigation of surgical tools, tracked by a localization system, in 2D X-ray images or a reconstructed 3D image acquired by an X-ray imaging system in a preferred coordinate system.

This invention relates to an imaging kit for a fluoroscopy-based navigation system. A fluoroscopy-based navigation system generally comprises an X-ray imaging system and a localization system configured to track the different trackers placed for example on the patient or on the surgical tools.

More in details the imaging kit of the present invention is configured to allow the acquisition of 2D images with an X-ray imaging system and the acquisition of data concerning the 3D spatial orientation and position of the trackers, said 2D images and data being suitable for being processed according to the steps of the method of the present invention.

An X-ray imaging system, generally used in a fluoroscopy-based navigation system, comprises at least one X-ray source and at least one X-ray detector. For example, the medical imaging system can be a C-arm, an O-arm or a scanner. A C-arm is designed to allow the source and detector to rotate along a C shaped gantry while obtaining projection images of the patient or object placed between the X-ray source and the X-ray detector of the gantry. The C-arm is capable of acquiring projection images over approximately 200° for example to be use for cone-beam computed tomography 3D image reconstruction.

The X-ray imaging system may be motorized, notably the C-shaped arm may comprise motors allowing movement horizontally, vertically and around the swivel axes, so that X-ray images of the patient are produced from almost any angle. Each motor is associated to an encoder allowing knowing, at any time, the relative position of the medical imaging system with respect to a reference position. When a 2D image is acquired, the corresponding position of the X-ray imaging system is recorded. Thus, each 2D image is recorded in the referential of the X-ray imaging system.

At least two images from two perpendicular views are acquired (for example anteroposterior and lateral) to allow a three-dimensional determination of the surgical tools' position. In general, several 2D images of the region of interest (i.e. the surgical field) are acquired at the beginning of the surgery and may be used for the reconstruction of a 3D image. Besides, during the surgical intervention, one or more extra 2D may be acquired and 3D images may be reconstructed in order to check the progress of the surgery. Usually in order to obtain a 3D, CT-like image, the C-arm system performs a sweep around the patient, acquiring up to several hundred 2D projections.

The localization system is usually a camera configured to track the trackers positioned on the patient and on the surgical tool. Therefore, the technology implemented by said camera depends on the type of trackers. The localization system is notably configured to track the position of the surgical tools tip so that the position and orientation of the surgical tools may be superimposed simultaneously on all acquired X-ray images or the 3D reconstructed image.

The imaging kit comprises a registration-tracking kit which comprises at least one registration phantom and a reference tracker.

The registration phantom comprises a fiducials support of substantially radiotransparent material comprising a plurality of radiopaque fiducials disposed in a known 3D position in a coordinate system of said registration phantom.

The radiopaque fiducials offer radiographic contrast relative to the fiducials support of substantially radiotransparent material and the body tissues. The radiopaque material may have a mass attenuation coeffect comprised in the range going from 1.0 cm²/g to 5.5 cm²/g for an energy of the X-ray beam comprised between 100 and 200 keV. The thickness of the fiducial may range from about 0.5 mm to about 10 mm and the radiopaque material may have at least one element with an atomic number of from about 22 to about 83. The fiducial may include an oxide or Salt material having at least one element with an atomic number of from about 22 to about 83. The fiducial may include barium Sulfate, bismuth trioxide, iodine, iodide, titanium oxide, Zirconium oxide, gold, platinum, Silver, tantalum, niobium, Stainless Steel, or combinations thereof. The fiducial may be coated or alloyed with a radiopaque material that has a mass attenuation coeffect comprised in the range between 1.0 cm²/g and 5.5 cm²/g for an energy of the X-ray beam comprised between 100 and 200 keV.

When a 2D image is acquired with the X-ray imaging system, the radiopaque fiducials are visible in the 2D image. Since the shape, size and arrangement of the radiopaque fiducials is known, the image can be located in the referential of the registration phantom and the reconstruction of the 3D image can be carried out using the position of the radiopaque fiducials in each 2D image for registration. The fiducials support is placed in the center of the surgical field, i.e. the center of the ROI, and the C-arm is placed so that the registration phantom lies in the central region of the field of view of the C-arm.

The radiopaque fiducials have a predetermined spatial arrangement which may be affected by a variability due to the uncertainty intrinsic to the fabrication. The exact 3D position in a coordinate system of said registration phantom of the radiopaque fiducials may be known, for example, by metrology or based on the assumption that the manufacturing process of the phantom is precise and reproducible enough to ensure that the desired position of the fiducials is obtained. Advantageously, the geometry of the phantom and the arrangement of the radiopaque fiducials are specific to the region of interest to be imaged.

The registration phantom is configured to be attached to the patient. In a preferred embodiment, the registration phantom is configured to be rigidly attached to a bone of the patient.

In one embodiment, the registration phantom is configured to be directly attached to a bone of the patients and to this end it comprises fixation means such as a bone screw, a plate, a nail, a pin, wires or the like. Alternatively, the registration phantom may be configured to be indirectly attached to a bone of the patients for example using attachment means external to the bone, such as an adhesive tape on the skin close to the bone, straps, etc. to immobilize the registration phantom with respect to the bone without passing through the patient's skin, or by open surgery using a clamp onto the bone (i.e. a clamp fixed on the spinous process).

According to one embodiment, the fiducials support comprises a central portion and two lateral wings extending on either side of said central portion. Preferably, the lateral wings are integral with the central portion. Depending on the application, the central portion and lateral wings may substantially extend in a plane; otherwise, the lateral wings may be sloping with respect to the central portion. In another embodiment, the wings may extend above the central portion. The fiducials support of the registration phantom may have any shape and size suitable for the intended application.

According to one embodiment, the fiducials in the fiducials support are disposed on a curved surface having a predefined curvature radius. In this embodiment, the central portion and lateral wings may substantially extend in a curved surface having a predefined curvature radius. Said curvature radius is indirectly related to the design of the C-arm of the X-ray imaging system (i.e. dimensions and distance between of the X-ray source and the X-ray detector and the trajectory used by the C-arm for the acquisition), and from the shape of the anatomical are of the patient that is under acquisition (i.e. curved surface of the back or the calf). The radius of curvature of the fiducials support is configured to conform to the curvature of the reconstructed volume of the 3D image acquired from the X-ray imaging device. Advantageously, this embodiment allows to optimize the position of the 3D volume to be reconstructed.

The reference tracker is configured to be attached to the patient. In a preferred embodiment, the reference tracker is configured to be rigidly attached to the patient.

In one embodiment, the reference tracker is configured to be directly attached to a bone of the patients and to this end it comprises fixation means such as a bone screw. In one alternative embodiment, the reference tracker may be indirectly attached to a bone of the patients for example using attachment means external to the bone, such as an adhesive tape on the skin close to the bone, straps, etc. to immobilize the reference tracker with respect to the bone without passing through the patient's skin, or by open surgery using a clamp onto the bone (i.e. a clamp fixed on the spinous process).

In one embodiment, the reference tracker and the registration phantom are independently rigidly fixed to one bone of the patient. Alternatively, the reference tracker and the registration phantom may be rigidly fixed each on a different bone of the patient. The reference tracker is configured to be placed in proximity of the registration phantom. Rigidly fixing the reference tracker and the registration phantom with respect to the patient, notably with respect to the body parts of the patient comprised in the ROI, allows to define a unique preferred coordinate system (i.e. referential) wherein the reference tracker, the registration phantom and the body parts of the patient comprised in the ROI has a fixed position and do not variate during time.

According to one embodiment, the tracker is an optical tracker (either active or passive). Alternatively, the tracker may be an electromagnetic tracker, which has the advantage of being more compact than an optical tracker.

In a preferred embodiment, the electromagnetic tracker contains inertial sensors that can be used to detect the presence of artefacts.

According to one alternative embodiment, the tracker is an ultrasound-based tracker or any other tracking technology known by the skilled person.

According to one embodiment, the reference tracker and the registration phantom forms a unique monoblock piece. In this embodiment, said unique monoblock piece comprises a fixation element to directly fixe it to a bone of the patient. When the monoblock registration-tracking kit of this embodiment is used to implement the method of the present invention, a preliminary calibration step may be done acquiring data on the 3D spatial orientation and position of the registration-tracking kit rigidly fixed onto the patient's bone. Additionally, manufacturing information concerning the relative position of the reference tracker and the registration phantom with respect to each other's may be used as well in said preliminary calibration step. The data (and manufacturing information) are used in preliminary calibration step to know the spatial position and orientation of the fiducials support and of the reference tracker in a unique preferred coordinate system. Since the registration-tracking kit is rigidly fixed onto the patient's bone said unique preferred coordinate system may be the coordinate system of the patient's bone. In this patient coordinate system, each point of the bone surface is fixed as well as the points defining the surface of the monoblock registration-tracking kit.

The imaging kit comprises at least one implantable element configured to be implanted inside a patient and at least one implant tracker. The implantable element and the implant tracker have each cooperating fixation means configured to movably attach the at least one implant tracker to the at least one implantable element.

The implantable element is an orthopedic implant manufactured to replace a missing joint or bone or to support a damaged bone. The implantable element may be plates, screws, nails, pins, wires and the like. The implantable element may be a permanent implant, which is expected to serve in the human body throughout the life span of the patients, or a temporary implant configured to be removed from the patient at the end of the surgery or after a predetermined period of time after the surgery for example equal to the time required to let bones heal.

Having an implant tracker directly attachable to an implantable element which is anyway meant to be fixed on a bone of the patient advantageously allows to avoid an invasive act of fixing, for example, one or more pins to the patient that has to be removed at the end of the surgery and which has potentially damaged the bone to which are fixed. Indeed, rigidly fixing the registration phantom and the reference tracker to a bone is fundament to have a fixed referential with respect to the patient. By "rigidly fixed" is meant in the present text that the base does not move with respect to the bone during the surgical intervention.

The reference tracker and the implant tracker are configured to be tracked by the localization system of the fluoroscopy-based navigation system.

According to one embodiment, the cooperating fixation means comprise complementary magnetic elements configured to maintain the at least one implant tracker and the at least one implantable element attached together. In this embodiment, the implant tracker is advantageously held in place magnetically so that if the implant tracker is accidentally hit by a member of the medical stuff it will pull out of the implantable element without damaging or displacing said implantable element from its initial fixation position on the patient's bone. Furthermore, thanks to this embodiment attachment and removal of the implant tracker can be made easily without requiring any tool.

Alternatively, the cooperating fixation means may be a clip-on fixation, a screw fixation or a clamp fixation.

According to one embodiment, each implant element is configured to carry one implant element.

According to one embodiment, the imaging kit comprises at least two implantable elements and one implant tracker for each implantable element. In this embodiment each implantable element is a pedicle screw configured to be implanted into a vertebra.

According to one embodiment, the implantable element is a joint prosthesis such as shoulder, hip, knee, ankle or finger prosthesis.

According to one embodiment, the imaging kit comprises at least two implantable elements, each implantable element being a bone screw configured to be implanted into a bone of a joint. This embodiment may be advantageously used for surgeries, such as joint replacement surgery, during which the relative positioning between the bones of a joint may change during the surgery.

According to one embodiment, the imaging kit further comprises a base made of a substantially radiotransparent material in order to be less visible as possible on the X-ray images. For substantially radiotransparent material it is meant a material having a small mass attenuation coeffect and that therefore does not quickly attenuate an X-ray beam passing through. More precisely a substantially radiotransparent material in the sense of the present invention has a mass attenuation coeffect inferior to 2.0·10⁻¹ cm²/g for an energy of the X-ray beam comprised between 100 and 200 keV. This definition of materials comprises for example the PMMA and polyethylene.

In one embodiment, the base is configured to be rigidly fixed to a patient's bone. The fixation may be either direct, using for example at least one percutaneous pin, needle, broach or screw implanted into the bone in a minimally invasive way, or indirect by using for example attachment means external to the bone, such as an adhesive tape on the skin close to the bone, straps, etc. to immobilize the base with respect to the bone without passing through the patient's skin, or by open surgery using a clamp onto the bone (i.e. a clamp fixed on the spinous process).

According to one embodiment, the base comprises at least one base fixation element, the registration phantom comprises at least one phantom fixation element and the reference tracker comprises a tracker fixation element, wherein said base fixation element, phantom fixation element and tracker fixation element are configured to attach, either separately or together, the reference tracker and the registration phantom to the base. This embodiment advantageously allows to have a modular registration-tracking kit wherein only the base is rigidly fixed on the patient's bone. In this way the registration phantom alone may be attached to the base only during acquisition of the 2D X-ray images and removed and replaced by the reference tracker during navigation.

According to one embodiment, the base has a support surface intended to face the bone, tissues surrounding the bone and/or the patient's skin. When the base is directly fixed to the bone, the patient's skin or tissues surrounding the bone may be located between the base and the bone. The base and the support surface may have any shape (e.g. circular, rectangular, etc.) and size suitable for the intended application, in particular depending on the shape and size of the body part to which the base has to be fixed. For example, for spine surgery, the base preferably has an elongated shape so as to be fixed to at least two or three adjacent vertebrae, whereas for shoulder surgery the base is rather circular with an oblong extension so as to be fixed to the acromion. The support surface may extend in a plane or may be either concave or convex, rigid or deformable.

Advantageously, the base may have a height of less than 20 mm so as to be very compact and only protrudes to a limited extent from the patient's skin. Thus, it is quite unlikely that the medical staff unintentionally hits the base and thus displaces it relative to the bone during the surgical intervention.

According to one embodiment, the base fixation element comprises at least one pin protruding from the surface of the base opposite to the support surface and the phantom fixation element comprises at least one respective complementary opening. Alternatively, the base fixation element comprises at least one opening in the surface of the base opposite to the support surface and the phantom fixation element comprises at least one respective complementary pin protruding.

According to one embodiment, the base fixation element and phantom fixation element comprise complementary magnetic elements configured to maintain the base and the registration phantom attached to each other. In this embodiment, the phantom fixation element is advantageously held in place magnetically so that if the registration phantom is accidentally hit by a member of the medical stuff it will pull out of the base without damaging the base fixation element or displacing the base from its initial fixation position on the patient's bone. Furthermore, thanks to this embodiment attachment and removal of the registration phantom and, if suitable, the tracker, can be made easily without requiring any tool.

According to one embodiment, the position and orientation of the registration phantom and the reference tracker attached to the base, either together either separately, is known in a unique preferred coordinate system. Said unique preferred coordinate system may be the coordinate system of the patient or equivanlently the coordinate system of the of the base of the registration-tracking kit since the base is rigidly fixed to the bone of the patient.

According to one embodiment, the base fixation element comprises at least one pin protruding from the surface of the base opposite to the support surface and the tracker fixation element comprises at least one respective complementary opening. Alternatively, the base fixation element comprises at least one opening in the surface of the base opposite to the support surface and the tracker fixation element comprises at least one respective complementary pin protruding.

According to one embodiment, the base fixation element and tracker fixation element comprise complementary magnetic elements configured to maintain the base and the registration phantom attached to each other.

In the embodiment of modular registration-tracking kit the fiducials support of the registration phantom may have any shape and size suitable for the intended application. In particular, since the registration phantom may be only attached to the base when it is required for image acquisition, the fiducials support can have size close to that of the field of view of the X-ray imaging system. This advantageously allows to have the radiopaque fiducials located at a greater distance from each other and thus improve the accuracy of the registration.

According to one embodiment, the central portion of the fiducials support bears the phantom fixation element.

According to one embodiment, the imaging kit comprises at least two registration phantoms, wherein the first registration phantom and the second registration phantom comprise cooperating fixation elements configured to attach the first registration phantom to the second registration phantom while being reproducibly fixed to the base.

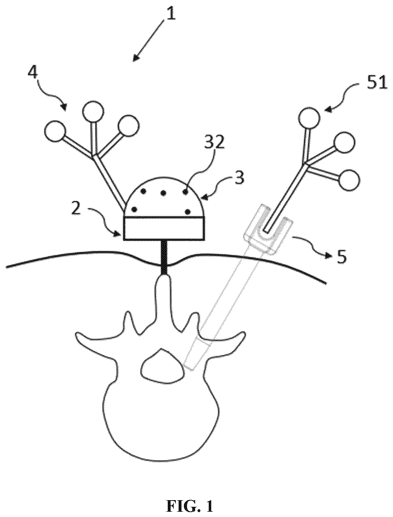

According to the embodiment of the invention illustrated in Figure 1, the registration-tracking kit 1 comprises a base 2 and a registration phantom 3. The base 2, made of a substantially radiotransparent material, is configured to be rigidly secured to a patient's bone, as shown in Figure 1. In this embodiment, the imaging kit comprises an implantable element 5 which is a pedicle screw implanted into the same vertebra to which is fixed the base 2. The implant tracker 51 is movably attached to the implantable element 5.

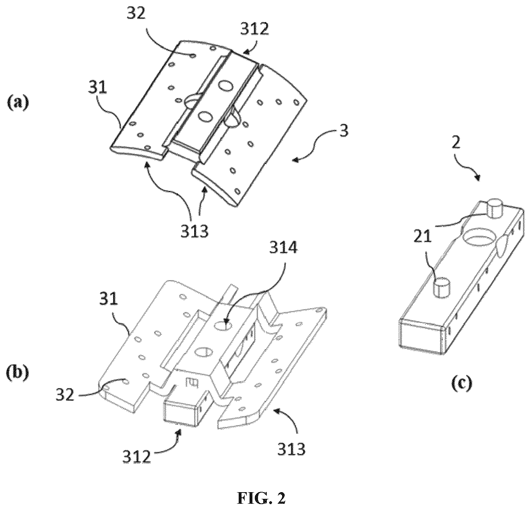

As illustrated in Figure 2a and 2b, the registration phantom 3 comprises a fiducials support 31 of substantially radiotransparent material comprising a plurality of radiopaque fiducials 32 disposed in a known 3D position in a coordinate system of said registration phantom 3. According to this embodiment illustrated in Figure 2c, the base 2 comprises a base fixation element 21 and the registration phantom 3 comprises a phantom fixation element 314 for reproducibly attaching the registration phantom 3 to the base 2.

Figures 2a and 2b show two prospective views of the rear and the front of the registration phantom 3. In this embodiment, the fiducials support 31 comprises a central portion 312 and two lateral wings 313 extending on either side of said central portion 312. In this embodiment, the wings 313 extends below the central portion 312 and have a curved surface. In this embodiment, the fiducial wings 313 comprise the radiopaque fiducials 32.

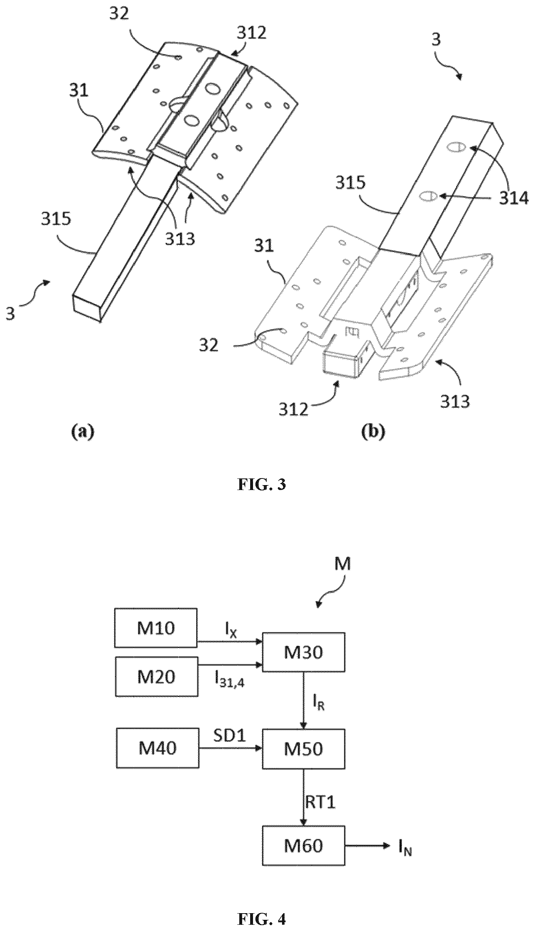

According to the embodiment illustrated in Figure 3a and 3b, the registration phantom 3 comprises an elongated member 315 extending from the fiducials support 31 along a longitudinal axis A. In this embodiment, the elongated member 315 bears the phantom fixation element 314. The elongated member 315 and the base fixation element 21 and phantom fixation element 314 are configured so as to provide at least one fixation position where the fiducials support 31 is deported with respect to the base 2. In this embodiment, the elongated member is directly fixed on the base and the elongated member fits the width and the length of the base.

The present invention relates to a method M for the generation of at least one navigable image I_{N} of at least one region of interest ROI of a patient for a fluoroscopy-based navigation system. Said fluoroscopy-based navigation system uses the imaging kit described in the embodiments above and a localization system 6 configured to track the reference tracker 4 and the implant tracker 51. The steps of the method M are illustrated by a block diagram in Figure 4.

The present method comprises a preliminary step M10 of receiving at least one set of 2D X-ray images Ix of at least one portion of the ROI.

The registration phantom is positioned so as to be at least partially comprised in the ROI during the acquisition of the images with the X-ray imaging system so that said acquired set of 2D images Ix comprises at least two 2D images containing each at least one detectable radiopaque fiducial of the registration phantom. In a preferred embodiment, as explained above, the registration phantom is placed in the center of the ROI and the C-arm is positioned so that the registration phantom is in the center of its field of view during the image acquisition. This configuration allows to obtain a large number of the images of the set of 2D images Ix containing each more than two detectable radiopaque fiducials of the registration phantom.

In order to have the registration phantom correctly position in the ROI during the acquisition of set of 2D images Ix the registration phantom had been previously rigidly attached to a patient bone.

The second step M20 comprises receiving information I_{31,4} on the position of the fiducials support 31 and the position of the reference tracker 4 in a patient coordinate system pcS fixed with respect to the patient's bone to which is attached the registration phantom. In other words, the information I_{31,4} comprises a list of coordinates allowing to univocally identify the exact position of the fiducials support and the reference tracker in one coordinate system: the patient coordinate system. Since the position of each fiducial is known in the coordinate system of the registration phantom and the position of the fiducials support is known in the patient reference system pcS, the position of each fiducial in the patient reference system pcS is calculated to be used in the registration step M30.

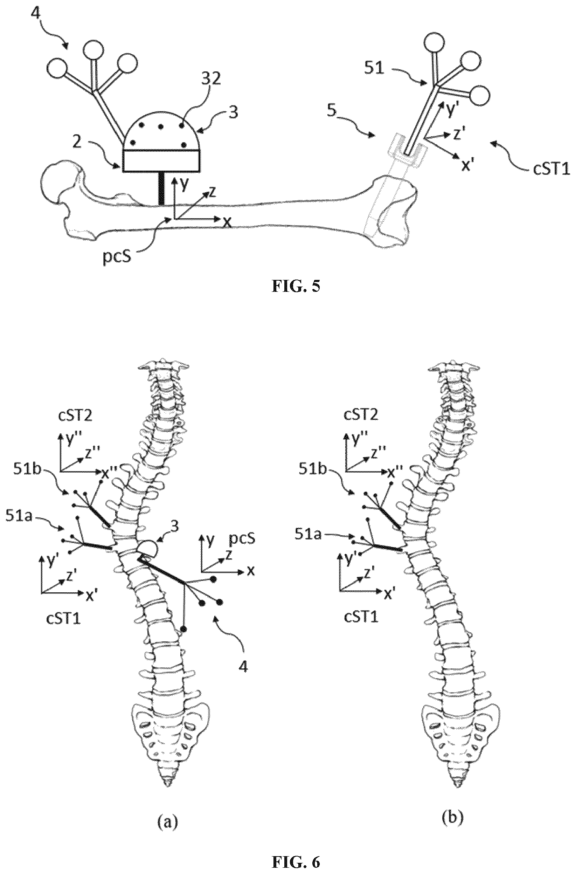

The fact that the patient coordinate system is fixed with respect to the patient's bone to which is attached the registration phantom means that each point of said bone's surface has a fixed position, that does not change during time, in the patient coordinate system pcS. Figure 5 schematically illustrates a possible example of patient coordinate system pcS having an x axis parallel to a longitudinal axis of the of a long bone.

According to embodiment wherein the reference tracker and the registration phantom are independently rigidly fixed to one or two different bones of the patient, a step of calibration using the localization system 6 is performed. During the calibration the localization system acquires information on the relative position and spatial orientation of the fiducials support 31 with respect to the reference tracker 4 so that their position may be calculated inside the unique referential of the patient coordinate system pcS.

In the embodiment wherein the reference tracker and the registration phantom form a monoblock, the relative position and spatial orientation of the fiducials support with respect to the reference tracker is known from the manufacturing designs and models. The information on the position of the fiducials support and the reference tracker in the patient coordinate system pcS is then calculated using the relative position and spatial orientation of the fiducials support and the reference tracker combined with information acquired by the localization system on the position and spatial orientation of the reference tracker when is rigidly attached to the bone of the patient.

In the embodiment wherein the registration-tracking system is modular, the relative position of the fiducials support with respect to the reference tracker when they are attached to the base, either together or separately, is known from the manufacturing designs and models. As before, an information is acquired with the localization system 6 on the position and spatial orientation of the reference tracker 4 when is attached to the base that is previously rigidly fixed to bone of the patient. The information I_{31,4} on the position of the fiducials support 31 and the reference tracker 4 in the patient coordinate system pcS is then calculated using the relative position and spatial orientation of the fiducials support and the reference tracker combined with information acquired by the localization system on the position and spatial orientation of the reference tracker when is rigidly attached to the bone of the patient. Since the base 2 is rigidly fixed to the bone of the patient it is also equivalent to refer to the position of the fiducials support and the reference tracker in a coordinate system of the base.

According to one embodiment, the method comprises the step of acquiring the set of 2D images I_{X} with the X-ray imaging system.

The method also comprises a step M30 of generating at least one registered image I_{R} by registering in the patient coordinate system at least one image of the set of 2D images I_{X}. The registration step is obtained using the known position of the fiducials support in said patient coordinate system.

According to one embodiment, the step M30 comprises first the registration of at least one image of the set of 2D images I_{X} in a coordinate system of the registration phantom. As described in the embodiment above the precise position of the fiducials in the coordinate system of the registration phantom is known. In the following description for "set of 2D images" it as to be understood at least one image of the set of 2D images.

In one embodiment, the step M30 further comprises:
- computing an optimal rigid transformation between a coordinate system of the X-ray system and the coordinate system of the registration phantom by optimizing the registration between the known 3D position of at least one fiducial of the registration phantom and the corresponding 2D position of said fiducial detected in at least two different images;
- applying said optimal rigid transformation to the 3D position of said at least one fiducial of the registration phantom to determine its respective transformed 3D position in the coordinate system of the X-ray imaging system; and
- registering the set of 2D images I_{X} in the patient coordinate system pcS using the known position of the fiducials support in the patient coordinate system pcS.

The method of the present invention comprises also the step M40 of receiving a first set of data SD1 obtained using the localization system.

Said first set of data SD1 comprises:
- data on position and 3D spatial orientation of the reference tracker 4 obtained using the localization system 6, wherein the reference tracker was previously attached to a bone of the patient in proximity of the registration phantom; and
- data on position and 3D spatial orientation of the at least one first implant tracker 51 when attached to the at least one first implantable element, wherein at least one first implantable element was previously rigidly fixed to a body part of the patient.

The reference tracker 4 and the first implantable element 51 may have been fixed on a same bone of the patient as shown in Figure 1 (i.e. vertebra) and 5 (i.e. femur) or two different bones.

The method comprises a step M50 of using the first set of data SD1 obtained from the localization system 6 to compute a first rigid transformation RT1 between the patient coordinate system pcS and a coordinate system of the at least one first implant tracker cST1. In this step the first set of data SD1 is used to calculate at least one rotation and one translation comprised in the first rigid transformation RT1.

Figure 5 shows a schematic representation of a possible coordinate system associated to the first implant tracker cST1. The cST1 coordinate system in this representation has a y'-axis parallel to a longitudinal axis of the first implantable element 51. In the cST1 coordinate system each point of the first implant tracker 51 is fixed thanks to the fact that the implantable element 5 is rigidly fixed to the bone.

The first rigid transformation RT1 advantageously allows to transform each point in each of the at least one registered image I_{R} from the patient coordinate system pcS to the coordinate system of the first implant tracker cST1.

The method comprises a step M60 of generating at least one navigable I_{N} image by applying the first rigid transformation RT1 to the at least one registered image I_{R} so that each point of the ROI in the at least one navigable image has a known position in the coordinate system of the at least one first implant tracker cST1. This advantageously allow the surgeon to navigate surgical tools in at least one navigable I_{N} image even after removing of the reference tracker 4 by using the first implant tracker 51.

According to one embodiment, the step M30 further comprises a step M31 of computing at least one 3D image within the patient coordinate system pcS using at least a part of the set of 2D images I_{X}. Whenever the step M30 comprises as well the step M31 of computing at least one 3D image, the at least one registered image I_{R} is a 3D reconstructed image registered in the patient coordinate system pcS. Equivalently, when step M31 is implemented, the at least one navigable image of step M60 is a navigable 3D image for which each point of the ROI represented in it has a known position in the coordinate system of the at least one first implant tracker cST1.

According to one embodiment, the set of 2D images comprises at least one 2D image being an anteroposterior view of the ROI and at least one 2D image being a lateral view of the ROI. Acquiring at least two perpendicular view of the ROI advantageously allows a correct three-dimensional determination of the surgical tools' position. This allows to reduce the number of images necessary for the navigation and therefore advantageously allows to reduce the amount of radiation received by the patient and the medical stuff during the X-ray image acquisition.

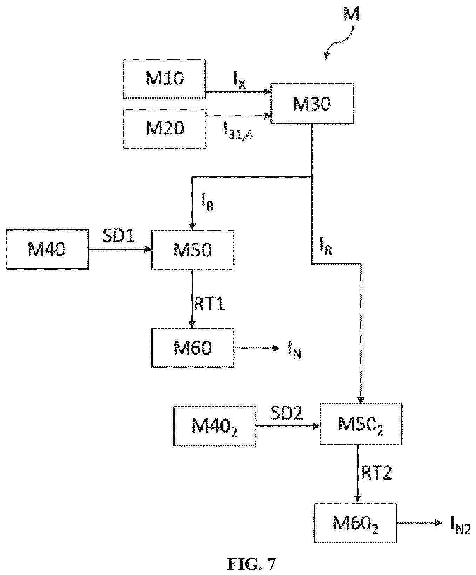

According to one embodiment, when a second implantable element 51b with at least one second implant tracker is present, as shown in Figure 6, the method further comprises multiples steps shown in the diagram bloc of Figure 7. According to this embodiment, the method comprises a step M40₂ receiving a second set of data SD2 obtained from the localization system when a second implant tracker 51b is attached to a second implantable element, wherein said second implantable element was previously fixed on a body part of the patient. Said second set of data SD2 comprises data on position and 3D spatial orientation of the reference tracker 51 and data on position and 3D spatial orientation of the second implant tracker 51b. In a step M50₂ said second set of data SD2 is used to compute a second rigid transformation RT2 between the patient coordinate system pcS and a coordinate system of the second implant tracker cST2. A step M60₂ is then implemented to generate at least one second navigable image I_{N2} by applying the second rigid transformation TR2 to the at least one registered image I_{R} of step M30 so that each point of the ROI in each image of the at least one second navigable image has a known position in the coordinate system of the at least one second implant tracker pcT2.

According to one embodiment, the imaging kit comprises more N implantable elements and at least N cooperating implant tracker, N being higher than 2. In this embodiment, the method comprises further steps in order to generate an i^{th} rigid transformation between the patient coordinate system and each coordinate system the i^{th} implant tracker pcTi. The method may be configured to generate N navigable images by applying the N i^{th} rigid transformation to the at least one registered image.

According to one embodiment, the method comprises a step M70 of receiving a user instruction selecting one among the first rigid transformation RT1 or the second rigid transformation RT2 to apply to the at least one registered image. If more than two implant trackers have been attached to the patient and N rigid transformation are calculated, the user may choose one among the N rigid transformation to apply to the at least one registered image. This embodiment advantageously allows the user to choose the more advantageous coordinate system with respect to the surgical gesture the has to be performed on the patient.

According to one embodiment, the method further comprises a step M71 of generating a dynamic correction of the at least one navigable image I_{N}. This step may be advantageously done when at least two implantable elements have been previously fixed on two different bones, notably when the relative position of these two bones may change during the surgery. Indeed, this dynamic correction step provides a correction in real time during the surgery of the navigable image in order to take into account any drift of the relative position between at least two implant trackers, and as consequence between the two bones to which are fixed through the implantable elements. This is notably the case for spine surgery where two vertebrae of the spine may change their relative position during the surgery, such as for example in the case of arthroplasty on a spinal disc or disc replacement, wherein an injured disc is replaced with an artificial one or spinal fusion which is used to join two vertebrae using for example rods and/or screws.

This embodiment may be advantageously implemented in the case arthroplasty on a spinal disc, by fixing a first and a second implantable element on two vertebrae adjacent to the disc to operate, as in Figure 6. In this case each vertebra is associated to a subregion of interest and a coordinate system of the implant tracker carried by the first and second implantable element. Therefore, even if the disc is alternated or removed, the navigable image I_{N} may be corrected in real time thanks to the definition of these independent subregions of interest.

This dynamic correction step may be directly implemented on the 2D navigable images I_{N} or on the 3D navigable image I_{N} obtained when the step M31 is implemented.

In one embodiment, the step M71 comprises selecting the first implant tracker as fixed reference. Step M71 further comprises defining a first subregion of interest comprising the first implant tracker and a second subregion of interest comprising the second implant tracker in the at least one navigable image I_{N}. In the case of multiple 2D navigable images I_{N} the first subregion and second subregion are defined for at least two images. Each point of the first subregion of interest, comprised the first implantable element and implant tracker, has a fixed position in a coordinate system cST1 of the first implant tracker. Equivalently, each point of the second subregion of interest, comprised the second implantable element and implant tracker, has a fixed position in a coordinate system cST2 of the second implant tracker. The subregions of interest may comprise a vertebra, part of a joint, a limb or the like. Rigidly fixing the implantable element carrying the implant tracker to an element (i.e. a bone) comprised in the subregion and being at least partially non-deformable, allows to fulfil the requirement for which each point of the subregion of interest must have a fixed position in the coordinate system of the implant tracker comprised in the subregion.

In one embodiment, the first subregion of interest and second subregions of interest are defined on the at least one first navigable image using a segmentation algorithm. Alternatively, the user may provide a manual input, such as for example a manual delineation of the contours of each subregion of interest.

According to one embodiment, the step M71 comprises receiving a third set of data obtained using the localization system. Said third set of data comprises data on position and 3D spatial orientation of the first implant tracker as a function of time and on position and 3D spatial orientation of the second implant tracker as a function of time. Said third set of data may be therefore used to compute a rigid transformation as a function of time between the coordinate system of the first implant tracker cST1 and the coordinate system of the second implant tracker pcT2.

The rigid transformation as a function of time may be applied to the second subregion of interest of the at least one first navigable image in order to obtain a dynamic correction as a function of time of the navigable image I_{N}(t) for any drift of the relative position between the first implant tracker 51a and the second implant tracker 51b.

In one embodiment wherein the first navigable image I_{N} is a set of 2D images, the first and second subregion are defined on at least two images of the at least one first navigable image I_{N} and therefore the rigid transformation as a function of time are applied on the second subregion defined on the at least two images of the at least one first navigable image.

In one embodiment wherein the first navigable image I_{N} is a 3D image, the first and second subregion are defined as two 3D sub-images of the 3D navigable image and therefore the rigid transformation as a function of time is applied on the 3D sub-image corresponding to the second subregion.

In one embodiment, the rigid transformation for a time *t* is only calculated when a change in the relative position between the first implant tracker and second implant tracker is detected. This change may be calculated using the third set of data.

The step M71 may be implement in an equivalent way by selecting the second implant tracker as fixed reference and therefore applying the rigid transformation as a function of time to the first subregion of interest of the at least one first navigable image.

According to one embodiment, each subregions of interest comprises a vertebra of the patient. In this embodiment, both the first and second implant element are pedicle screws previously fixed to each of the vertebra of the patient, as in Figures 6 and 7.

According to one embodiment, each subregions of interest comprises each a portion of a joint of the patient, preferably each subregion corresponds to at least a portion of one bone of a joint. For example, the first subregion may comprise a portion of the femur and the second subregion may comprise a portion of the tibia or fibula. In this embodiment, the first and second implantable elements are bone screws configured to be fixed to said portions of the joint.

According to the embodiment wherein registration-tracking kit 1 is modular and comprises a base, the method of the present inventions comprises a first step of receiving at least one set of 2D X-ray images Ix of at least one portion of the region of interest, wherein the registration phantom 3 is attached on the base 2 during the acquisition of the set of 2D X-ray images, said base being previously fixed to a patient's bone, such that said set of 2D images comprises at least two 2D images containing each at least one detectable radiopaque fiducial of the registration phantom. The position of fiducials support in the coordinate system of the base is known.

According to this embodiment, the method comprises a step of generating at least one registered image I_{R} by registering the set of 2D images Ix in the coordinate system of the base using the known position of the fiducials support in the coordinate system of the base.

In this embodiment, the method further receives a first set of data SD1 obtained using the localization system 6, wherein during the acquisition of said first set of data the reference tracker 4 is attached to the base 2 and the at least one first implant tracker 51 is attached to the at least one first implantable element 5, previously rigidly fixed to a body part of the patient. Therefore, said first set of data comprises data on orientation and 3D spatial orientation of the reference tracker attached on the base and data on orientation and 3D spatial orientation of the at least one first implant tracker attached to the at least one first implantable element.

Finally, according to this embodiment, the first set of data SD1 is used to compute a first rigid transformation RT1 between the coordinate system of the base and a coordinate system of the at least one first implant tracker and generate at least one navigable image I_{N} by applying the first rigid transformation RT1 to the at least one registered image I_{R} so that each point of the ROI in the navigable images has a known position in the coordinate system of the at least one first implant tracker.

The method for generating navigable images of at least one ROI of a patient for a fluoroscopy-based navigation system may as well be performed according to the steps that follows.

First step may comprise providing an imaging kit according to any one of the embodiments hereabove and fixing the registration phantom 3 and the reference tracker 4 of the registration-tracking kit 1 to a body part of the patient in proximity of the region of interest.

In case of modular registration-tracking kit, only the base 2 may be first rigidly fixed on the body part of the patient in proximity of the ROI and after at least one registration phantom 3 may be attached onto the base using the base fixation element and the phantom fixation element. In this case, the position of the fiducials support is known in a coordinate system of the base.

Once the registration phantom has been correctly positioned in the ROI and the X-ray imaging system has been positioned so that the registration phantom is at least partially comprised in its field-of-view (preferably in the center of its field-of-view), the X-ray imaging system may be used to acquire at least one set of 2D images of the ROI, wherein at least two image of the set of 2D images contain each at least one detectable radiopaque fiducial of the registration phantom.

In this specific embodiment, the method comprises a step of receiving information on the position of the fiducials support and the position of the reference tracker in a patient coordinate system pcS fixed with respect to the patient's bone to which is attached the registration phantom. Then, at least one registered image I_{R} may be generated by registering the set of 2D images I_{X} in the patient coordinate system pcS using the known position of the fiducials support 31 in said patient coordinate system.

In case of modular registration-tracking kit, the information I_{31,4} comprises the position of the fiducials support 31 and the position of the reference tracker 4 in a coordinate system of the base. Then, at least one registered image I_{R} may be generated by registering the set of 2D images I_{X} in the coordinate system of the base using the known position of the fiducials support 31 in the coordinate system of the base. In this case, the registration phantom 3 may be removed from the base and the reference tracker 4 may be attached to the base 2 using the base fixation element and the tracker fixation element.

The localization system 6 may be associated to the reference tracker 4.

A step may further comprise rigidly fixing the at least one implantable element 5 of the imaging kit to a body part of the patient in proximity of the region of interest and attaching the at least one implant tracker 51 onto the at least one implantable element 5. The reference tracker 4 may be used to navigate surgical tools during the fixation of the at least one implantable element 5 to the patient's bone.

The localization system 6 may be further configured to be associated to the implant tracker 51 attached to the implantable element 5. The localization system 6 acquires a first set of data SD1, obtained, comprising data on position and 3D spatial orientation of the reference tracker, and position and 3D spatial orientation of the at least one first implant tracker attached to the at least one first implantable element.

Then, the first set of data SD1 obtained from the localization system may be used to compute a rigid transformation RT1 between the patient coordinate system pcS and a coordinate system of the implant tracker cST1.

In case of modular registration-tracking kit, the rigid transformation is calculated between the coordinate system of the base and a coordinate system of the implant tracker cST1.

In a following step, the first rigid transformation RT1 may be applied to the at least one registered image I_{R} so as to generate at least one navigable image I_{N} wherein each point of the ROI in said at least one navigable image has a known position in the coordinate system of the at least one first implant tracker cST1.

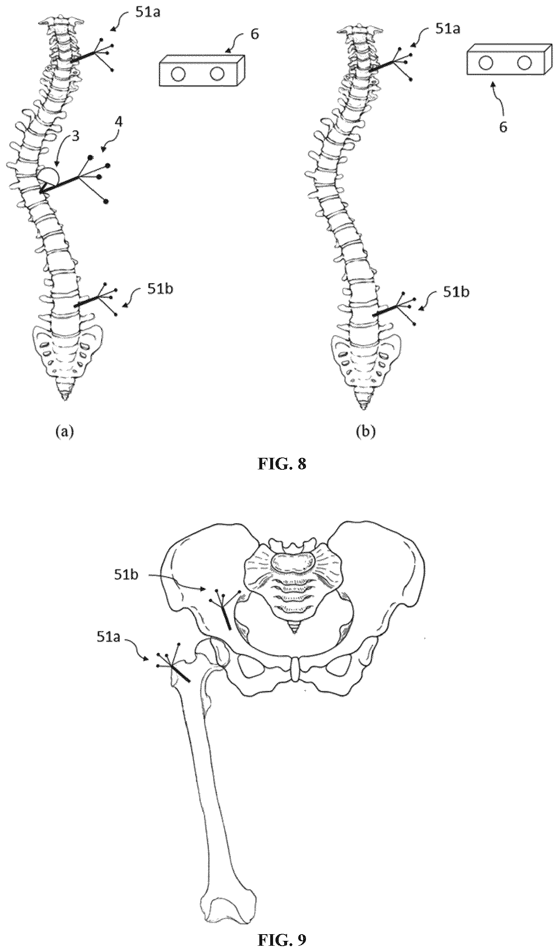

Finally, since each point of the ROI in the navigable image I_{N} has a known position in the coordinate system of first implant tracker cST1 is then possible to remove the base, the reference tracker and the registration phantom from the patient. This final step is illustrated in Figures 6b and 8b, where the elements of the registration-tracking kit 1 are removed and only the first and second implantable element with the related implant trackers are left for navigation.

The present invention relates to a data processing system for generating navigable images of at least one region of interest ROI of a patient for a fluoroscopy-based navigation system according to the method described hereabove.

The data processing system generated navigable images I_{N} for a fluoroscopy-based navigation system comprising an X-ray imaging system and a localization system using the imaging kit according to the present invention. Said localization system is configured to track the reference tracker 4 and the at least one implant tracker 51 of the imaging kit of the present invention.

In one embodiment, the data processing system comprises an input module configured to receive at least one set of 2D X-ray images I_{X} of at least one portion of the ROI. Said set of 2D X-ray images I_{X} has been previously acquired while the registration phantom 3 was attached to a patient's bone. Notably, the registration phantom 3 was placed in the center of the ROI and the X-ray imaging system was positioned so that the registration phantom 3 is at least partially comprised in the ROI during the acquisition of the set of 2D X-ray images I_{X} in order to obtain a set of 2D images comprising at least two 2D images having each at least one detectable radiopaque fiducial of the registration phantom 3. The input module is further configured to receive information on the position of the fiducials support 31 and the reference tracker 4 in a patient coordinate system pcS fixed with respect to the patient's bone to which is attached the registration phantom 3.

In one embodiment, the data processing system comprises a data storage medium comprising the 3D spatial position of the fiducials support 31 and the fiducials 32 in the coordinate system of the registration phantom. Since the patient's bone to which is rigidly attached the registration phantom 3 has a fixed position in the patient coordinate system pcS also the registration phantom 3 has a fixed position in the patient coordinate system pcS. As consequence, it is possible to precisely calculate the position of the fiducials support in the patient reference system pcS.

In one embodiment, the data storage medium comprises a preliminary information the relative position and spatial orientation of the fiducials support 31 with respect to the reference tracker 4.

According to embodiment wherein the reference tracker 4 and the registration phantom 3 are independently rigidly fixed to one same bone or two different bones of the patient, said preliminary information is obtained during a calibration step wherein the localization system 6 acquires information on the relative position and spatial orientation of the fiducials support with respect to the reference tracker 4 so that their position may be calculated inside the unique referential of the patient coordinate system pcS.

In the embodiment wherein the reference tracker 4 and the registration phantom 3 form a monoblock, the preliminary information is known from the manufacturing designs and models.

In the embodiment wherein the registration-tracking kit is modular, the preliminary information is known from the manufacturing designs and models.

Both for the monoblock and modular registration kit, the information on the position of the fiducials support 31 and the reference tracker 4 in the patient coordinate system pcS may be then calculated by the data processing system using the relative position and spatial orientation of the fiducials support and the reference tracker combined with information acquired by the localization system 6 on the position and spatial orientation of the reference tracker when is rigidly attached to the bone of the patient.

In one embodiment, the data processing system comprises a registration module configured to generate at least one registered image I_{R} by registering the set of 2D images in the patient coordinate system pcS using the known position of the fiducials support 31 in the patient coordinate system pcS.

According one embodiment, the registration module is configured to first register at least one image of the set of 2D images in a coordinate system of the registration phantom. As described in the embodiment above, the precise position of the fiducials in the coordinate system of the registration phantom is known. As above for "set of 2D images" it as to be understood at least one image of the set of 2D images. The registration module further computes an optimal rigid transformation between a coordinate system of the X-ray system and the coordinate system of the registration phantom by optimizing the registration between the known 3D position of at least one fiducial of the registration phantom and the corresponding 2D position of said fiducial detected in at least two different 2D images. The following step implemented by this module is applying said optimal rigid transformation to the 3D position of said at least one fiducial of the registration phantom to determine its respective transformed 3D position in the coordinate system of the X-ray imaging system. Finally, the last step of the registration module comprises registering the set of 2D images in the patient coordinate system using the known position of the fiducials support in the patient coordinate system.

According to one embodiment, the registration module is further configured to compute at least one 3D image within the patient coordinate system pcS using at least a part of the set of 2D images.

According to one embodiment, the data processing system comprises a transformation calculation module configured to generate at least one first navigable image I_{N} thanks to the implementation of at least three steps.

The first step comprises receiving a first set of data SD1 obtained using the localization system 6. Said first set of data SD1 comprises data on orientation and 3D spatial orientation of the reference tracker 4 and the at least one first implant tracker 51 attached to the at least one first implantable element 5. The data obtained using the localization system 6 have been acquired while the reference tracker 4 was attached to a bone of the patient in proximity of the registration phantom 3 and the at least one first implantable element 5 was rigidly fixed to a body part of the patient.

As described previously for the method, the reference tracker 4 and the first implantable element 5 may be fixed on the same bone in two different fixation point, as in Figure 5 or on two different bones, for example belonging to one joint (Fig. 9).

The second step comprises using the first set of data SD1 obtained from the localization system 6 to compute a first rigid transformation RT1 between the patient coordinate system pcS and a coordinate system of the at least one first implant tracker cST1.

The third set comprises generating at least one first navigable image I_{N} by applying the first rigid transformation RT1 to the at least one registered image I_{R} so that each point of the ROI in the at least one first navigable image I_{N} has a known position in the coordinate system of the at least one first implant tracker cST1.

Once the first navigable image I_{N} is transformed in the coordinate system of the first implant tracker cST1there is no more need for the reference tracker 4 that may be removed as well as the registration phantom 3 to perform the surgery. Therefore, the method of the present invention advantageously allows to provide navigable image I_{N} to be navigated by any surgical tool without the need of a cumbersome reference on the patient.

According to one embodiment, the transformation calculation module is further configured to iteratively perform the three steps described hereabove for each implant tracker being attached to at least one implantable element fixed on a bone of the patient.

For example, the transformation calculation module may be further configured to:
- receive a second set of data SD2 obtained from the localization system 6 with a second implant tracker 51b attached to a second implantable element, previously fixed on a body part of the patient, said second set of data SD2 comprising data on position and 3D spatial orientation of the reference tracker 4 and 3D spatial orientation of the second implant tracker 51b;
- use the second set of data SD2 obtained from the localization system to compute a second rigid transformation RT2 between the patient coordinate system pcS and a coordinate system of the second implant tracker pcT2;
- generate at least one second navigable image I_{N2} by applying the second rigid transformation RT2 to the at least one registered image I_{R} so that each point of the ROI in the at least one second navigable image has a known position in the coordinate system of the at least one second implant tracker pcT2.

According to one embodiment, the data processing system further comprises a visualization module configured to receive a user instruction selecting one among the first rigid transformation TR1 or the second rigid transformation TR2 to be applied to the at least one registered image. These embodiments advantageously allow the simultaneous representation of multiple, anatomically independent ROIs of the patient using the navigable image I_{N}. Indeed, as show in Figures 6 and 8, depending on the type of surgery the implant elements carrying the implant trackers may be fixed on to adjacent bones or to distant ones.

According to on embodiment, the data processing system further comprises a dynamic image correction module configured to be used when at least two implantable elements, each carrying at least one implant tracked, are fixed on two different bones of the patient, as in Figure 6, 8 or 9.

Indeed, during fluoroscopy-based navigation surgery the X-ray 2D images are usually acquired only once during the surgery. Therefore, the modifications of the anatomy of the ROI, which may have been induced by the surgery itself, are not taken into account for the navigation. This may cause, for example, an incorrect placement of bone screw into the patient. The dynamic image correction module advantageously allows to correct for these modifications of the anatomy of the ROI in order to truly represent the current anatomy of the ROI.

In one embodiment, the dynamic image correction module is configured to:
- select the first implant tracker 51a as fixed reference;
- define a first subregion of interest comprising the first implant tracker 51a and a second subregion of interest comprising the second implant tracker 51b for the at least one first navigable image;
- receiving a third set of data obtained using the localization system comprising data on position and 3D spatial orientation of the first implant tracker and the second implant tracker as a function of time;
- use the third set of data to compute a rigid transformation as a function of time between the coordinate system of the first implant tracker cST1 and the coordinate system of the second implant tracker pcT2;
- apply the rigid transformation as a function of time to the second subregion of interest of the at least one first navigable image so as to dynamically correct during time said navigable image for any drift of the relative position between the first implant tracker and the second implant tracker.

As described above, the first step may be equivalently the selection of the second implant tracker 51b as fixed reference.

According to one embodiment, the dynamic image correction module is further configured to automatically define the first and second subregions of interest on the at least one first navigable image using a segmentation algorithm.

Alternatively, the dynamic image correction module may be configured to receive, as a manual input of the user, the definition of the first and second subregions of interest, for example as a manual delineation of the contours of each subregion of interest.

According to one embodiment, each subregions of interest comprises a vertebra of the patient, and the first and second implant element are pedicle screws fixed to each of the vertebra as shown in Figures 6 and 8.

According to one embodiment, each subregions of interest comprises each a portion of a joint of the patient, and the first and second implantable elements are bone screws configured to be fixed to said portions of the joint. The specific case of a hip joint is illustrated in Figure 9, where one first implantable element with the first implant tracker 51a is fixed on the femur and one second implantable element the second implant tracker 51b is fixed on the pelvis of a patient.

According to one embodiment, the set of 2D images received by the input module comprises at least one 2D image being a anteroposterior view of the ROI and at least one 2D image being a lateral view of the ROI.

According to one embodiment, further comprising a navigation module configured to navigate in the ROI at least one surgical tool comprising a tracker with the at least one navigable image. The tracker of the surgical tool is associated with the localization system 6.

The present method and data processing system advantageously allow to remove from the surgical field both the reference tracker and the registration phantom that can obstruct the gestures of the surgeon, the movements of an automated surgical robot arm or mask surgical entry points during minimally invasive or open surgeries thanks to the use of implant trackers attached on one or more implant elements that would had been fixed to the patient anyway during the surgery. Fixing implant trackers on the implantable elements instead on fixing them directly on the patient allows to reduce the intrusive actions done on the patient and therefore reduce the number of unnecessary lesions caused during the surgery.

The present invention also relates to a computer program comprising instructions, which when the program is executed by a computer, causes the computer to carry out the steps of the method described above.

The computer program product to perform the method as described above may be written as computer programs, code segments, instructions or any combination thereof, for individually or collectively instructing or configuring the processor or computer to operate as a machine or special-purpose computer to perform the operations performed by hardware components. In one example, the computer program product includes machine code that is directly executed by a processor or a computer, such as machine code produced by a compiler. In another example, the computer program product includes higher-level code that is executed by a processor or a computer using an interpreter. Programmers of ordinary skill in the art can readily write the instructions or software based on the block diagrams and the flow charts illustrated in the drawings and the corresponding descriptions in the specification, which disclose algorithms for performing the operations of the method as described above.

The present invention also relates to a computer-readable storage medium comprising instructions that when executed by a computer, causes the computer to carry out the steps of the method.

According to one embodiment, the computer-readable storage medium is a non-transitory computer-readable storage medium.

Computer programs implementing the method of the present embodiments can commonly be distributed to users on a distribution computer-readable storage medium such as, but not limited to, an SD card, an external storage device, a microchip, a flash memory device, a portable hard drive and software websites. From the distribution medium, the computer programs can be copied to a hard disk or a similar intermediate storage medium. The computer programs can be run by loading the computer instructions either from their distribution medium or their intermediate storage medium into the execution memory of the computer, configuring the computer to act in accordance with the method of this invention. All these operations are well- known to those skilled in the art of computer systems.

The instructions or software to control a processor or computer to implement the hardware components and perform the methods as described above, and any associated data, data files, and data structures, are recorded, stored, or fixed in or on one or more non-transitory computer-readable storage media. Examples of a non-transitory computer-readable storage medium include read-only memory (ROM), random-access memory (RAM), flash memory, CD- ROMs, CD- Rs, CD+ Rs, CD- RWs, CD+ RWs, DVD- ROMs, DVD- Rs, DVD+ Rs, DVD- RWs, DVD+ RWs, DVD- RAMs, BD- ROMs, BD- Rs, BD- R LTHs, BD- REs, magnetic tapes, floppy disks, magneto-optical data storage devices, optical data storage devices, hard disks, solid-state disks, and any device known to one of ordinary skill in the art that is capable of storing the instructions or software and any associated data, data files, and data structures in a non-transitory manner and providing the instructions or software and any associated data, data files, and data structures to a processor or computer so that the processor or computer can execute the instructions. In one example, the instructions or software and any associated data, data files, and data structures are distributed over network-coupled computer systems so that the instructions and software and any associated data, data files, and data structures are stored, accessed, and executed in a distributed fashion by the processor or computer.

While various embodiments have been described and illustrated, the detailed description is not to be construed as being limited hereto. Various modifications can be made to the embodiments by those skilled in the art without departing from the true spirit and scope of the disclosure as defined by the claims.

## Claims

1. An imaging kit for a fluoroscopy-based navigation system including a localization system, said imaging kit comprising:
- a registration-tracking kit (1) comprising:
• at least one registration phantom (3) comprising a fiducials support (31) of substantially radiotransparent material having a plurality of radiopaque fiducials (32) and being configured to be attached to a bone a patient;
• a reference tracker (4) being configured to be attached to a bone the patient;
- at least one implantable element (5) configured to be implanted inside a patient and at least one implant tracker (51), the implantable element (5) and the implant tracker (51) having each cooperating fixation means configured to movably attach the at least one implant tracker (51) to the at least one implantable element (5);
wherein the reference tracker (4) and the implant tracker (51) are configured to be tracked by the localization system.

2. The imaging kit according to claim **1,** wherein the cooperating fixation means comprise complementary magnetic elements configured to maintain the at least one implant tracker (51) and the at least one implantable element (5) attached together.

3. The imaging kit according to either one of claims **1** or **2,** the registration-tracking kit (1) further comprising a base (2) made of a substantially radiotransparent material and being configured to be rigidly fixed to a bone of the patient; wherein said base (2) comprises at least one base fixation element (21), the registration phantom (3) comprises at least one phantom fixation element (314) and the reference tracker (4) comprises a tracker fixation element, said base fixation element (21), phantom fixation element (314) and tracker fixation element being configured to attach, either separately or together, the reference tracker (4) and the registration phantom (3) to the base (2).

4. The imaging kit according to any one of claims **1** to **3,** comprising two implantable elements (5) and one implant tracker (51) for each implantable element (5), wherein each implantable element (5) is a pedicle screw configured to be implanted into a vertebra.

5. The imaging kit according to any one of claims **1** to **3,** wherein the implantable element (5) is a joint prosthesis.

6. The imaging kit according to any one of claims **1** to **3,** comprising at least two implantable elements (5), each implantable element being a bone screw configured to be implanted into a bone of a joint.

7. Method for generating of navigable images of at least one region of interest ROI of a patient for a fluoroscopy-based navigation system using an X-ray imaging system, an imaging kit according to any one of claims **1** to **6** and a localization system (6) configured to track the reference tracker (4) and the at least one implant tracker (51) of the imaging kit, said method comprising the following steps:
a) receiving (M10) at least one set of 2D X-ray images (Ix) of at least one portion of the ROI, wherein the registration phantom (3) had been previously attached to a patient's bone so that the registration phantom is at least partially comprised in the ROI during the acquisition of the set of 2D X-ray images, such that said set of 2D images comprises at least two 2D images containing each at least one detectable radiopaque fiducial of the registration phantom (3);
b) receiving (M20) information (I_{31,4}) on the position of the fiducials support (31) and the position of the reference tracker (4) in a patient coordinate system (pcS) fixed with respect to the patient's bone to which is attached the registration phantom;
c) generating (M30) at least one registered image (I_{R}) by registering the set of 2D images in the patient coordinate system (pcS) using the known position of the fiducials support (31) in said patient coordinate system (pcS);
d) receiving (M40) a first set of data (SD1), obtained using the localization system, comprising data on position and 3D spatial orientation of the reference tracker, said reference tracker previously attached to a bone of the patient in proximity of the registration phantom, and position and 3D spatial orientation of the at least one first implant tracker attached to the at least one first implantable element, said at least one first implantable element previously rigidly fixed to a body part of the patient;
e) using (M50) the first set of data obtained from the localization system (6) to compute a first rigid transformation (RT1) between the patient coordinate system (pcS) and a coordinate system of the at least one first implant tracker (cST1);
f) generating (M60) at least one navigable image (I_{N}) by applying the first rigid transformation to the at least one registered image (I_{R}) so that each point of the ROI in the at least one navigable image has a known position in the coordinate system of the at least one first implant tracker (cST1).

8. A data processing system for generating navigable images of at least one region of interest ROI of a patient for a fluoroscopy-based navigation system using an X-ray imaging system, an imaging kit according to any one of claims **1** to **6** and a localization system, said localization system (6) being configured to track the reference tracker and the at least one implant tracker of the imaging kit, said data processing system comprising:
- an input module configured to receive at least one set of 2D X-ray images (Ix) of at least one portion of the ROI, wherein the registration phantom (3) had been previously attached to a patient's bone so that the registration phantom (3) is at least partially comprised in the ROI during the acquisition of the set of 2D X-ray images, such that said set of 2D images comprises at least two 2D images containing each at least one detectable radiopaque fiducial of the registration phantom (3); said input module being further configured to receive information (I_{31,4}) on the position of the fiducials support (31) and the reference tracker (4) in a patient coordinate system (pcS) fixed with respect to the patient's bone to which is attached the registration phantom (3);
- a registration module configured to generate at least one registered image (I_{R}) by registering the set of 2D images in the patient coordinate system (pcS) using the known position of the fiducials support (31) in the patient coordinate system (pcS);
- a transformation calculation module configured to:
• receive a first set of data (SD1) obtained using the localization system (6) comprising data on position and 3D spatial orientation of the reference tracker (4), being previously attached to a bone of the patient in proximity of the registration phantom (3), and data on position and 3D spatial orientation of the at least one first implant tracker (51) attached to the at least one first implantable element (5), said the at least one first implantable element (5) being previously rigidly fixed to a body part of the patient;
• use the first set of data to compute a first rigid transformation (RT1) between the patient coordinate system (pcS) and a coordinate system of the at least one first implant tracker (cST1);
• generate at least one first navigable image (I_{N}) by applying the first rigid transformation (RT1) to the at least one registered image (I_{R}) so that each point of the ROI in the at least one first navigable image (I_{N}) has a known position in the coordinate system of the at least one first implant tracker (cST1).

9. The data processing system according to claim **8,** wherein the registration module is further configured to compute at least one 3D image within the patient coordinate system (pcS) using at least a part of the set of 2D images.

10. The data processing system according to either one of claim **8** or **9,** wherein the transformation calculation module is further configured to:
- receive a second set of data (SD2) obtained from the localization system (6) with a second implant tracker attached to a second implantable element, previously fixed on a body part of the patient, said second set of data comprising data on position and 3D spatial orientation of the reference tracker and 3D spatial orientation of the second implant tracker;
- use the second set of data (SD2) obtained from the localization system (6) to compute a second rigid transformation (RT2) between the patient coordinate system (pcS) and a coordinate system of the second implant tracker (pcT2);
- generate at least one second navigable image (I_{N2}) by applying the second rigid transformation to the at least one registered image so that each point of the ROI in the at least one second navigable image has a known position in the coordinate system of the at least one second implant tracker (pcT2).

11. The data processing system according to claim **10,** further comprises a visualization module configured to receive a user instruction selecting one among the first rigid transformation (RT1) or the second rigid transformation (RT2) to be applied to the at least one registered image (I_{R}).

12. The data processing system to claim **10,** further comprising a dynamic image correction module configured to:
- select the first implant tracker as fixed reference;
- define a first subregion of interest comprising the first implant tracker and a second subregion of interest comprising the second implant tracker for the at least one first navigable image;
- receiving a third set of data obtained using the localization system (6) comprising data on position and 3D spatial orientation of the first implant tracker and the second implant tracker as a function of time;
- use the third set of data to compute a rigid transformation as a function of time between the coordinate system of the first implant tracker (cST1) and the coordinate system of the second implant tracker (pcT2);
- apply the rigid transformation as a function of time to the second subregion of interest of the at least one first navigable image so as to dynamically correct during time said navigable image for any drift of the relative position between the first implant tracker and the second implant tracker.

13. The data processing system according to claim **12,** wherein the first and second subregions of interest are defined on the at least one first navigable image using a segmentation algorithm or by a manual input of the user.

14. The data processing system according to any one of claims **10** to **13,** wherein each subregions of interest comprises a vertebra of the patient, and the first and second implant element are pedicle screws fixed to each of the vertebra.

15. The data processing system according to any one of claims **10** to **13,** wherein each subregions of interest comprises each a portion of a joint of the patient, and the first and second implantable elements are bone screws configured to be fixed to said portions of the joint.

16. The data processing system to any one of claims **8** to **15,** wherein the set of 2D images comprises at least one 2D image being an anteroposterior view of the ROI and at least one 2D image being a lateral view of the ROI.

17. The data processing system to any one of claims **8** to **16,** further comprising a navigation module configured to navigate at least one surgical instrument, comprising a tracker, in the ROI using the at least one navigable image (I_{N}).
